# EUROPEAN PATENT SPECIFICATION

(11) **EP 0 747 037 B1**
(45) Date of publication and mention of the grant of the patent: **06.11.2002**
(21) Application number: 95201511.3
(22) Date of filing: 08.06.1995
(51) Int. Cl.: A61K 7/16

(54) **Mouth-care products**
Mittel zur orale Hygiene
Compositions pour les soins buccaux

(43) Date of publication of application: 11.12.1996
(73) Proprietor: Sara Lee/DE N.V., 3532 AA Utrecht (NL)
(72) Inventor: Harms, Johan Gerard, 2871 PB Schoonhoven (NL)
(74) Representative: Smulders, Theodorus A.H.J., Ir.

(56) References cited:
- EP-A- 0 391 803
- EP-A- 0 437 360
- US-A- 4 992 259

## Description

The present invention is related to a toothpaste containing microspheres.

From EP-A 437,360 it is known to use hydrocolloid bulking agents, or hydrocolloid microspheres of particle size between 1.0 and 200 um in various products including toothpaste's. These microspheres are prepared by mixing a solution of a calcium salt and an alginate solution. Although the document cites the use of the microspheres in toothpaste, there is no working example or other information provided in the document about the actual use in toothpaste.

In practice it has been found, that the use of the microspheres as prepared in accordance with the teachings of this document in a toothpaste, does not result in a suitable material, as the stability of the microspheres is insufficient for this application.

It is an object of the invention to provide a toothpaste containing microspheres, which toothpaste and microspheres are sufficiently stable to be stored for prolonged periods of time, for example up to six months, under ambient conditions.

Further it is an object of the present invention to provide novel types of toothpaste's having novel and advantageous functions.

According to the invention these and other objects are obtained by the provision of a mouth-care product in the form of a toothpaste comprising microspheres having a particle size in the range of 0.1 to 4 mm, said microspheres being based on zinc-stabilised alginate.

Surprisingly it has been found that microspheres based on zinc-stabilised alginate possess the necessary stability in the said particle size range, the latter being advantageous in view of the properties of the toothpaste, such as appearance.

Preferably, the microspheres contain at least one pigment.

The properties, especially the stability of the microspheres, are further improved if the toothpaste contains complexed calcium and/or no free fluoride ions.

Preferably any calcium present in the toothpaste is in the form of complexed calcium, such as in the form of calcium citrate, whereas the fluoride is preferably supplied in the form of fluor-phosphate compounds, such as mono-fluorophosphate.

The presence of fluoride providing agents in the mouth-care products according to the present invention is important. The use of fluoride ions, or fluor-ion providing compounds as anti-caries agents is well known in the art. The amount of fluoride-providing compounds is dependent to some extent upon the type of compound, its solubility, and the type of oral preparation, but it must be a non toxic, effective amount, generally about 0.005 to about 3.0% in the preparation, e.g. gel or tooth paste. In the case of sodium monofluorophosphate (MFP), the compound may be present in an amount of about 0.1-3 wt.%. When using MFP as a fluor compound it is preferred for the toothpaste, for stability reasons, to have a pH of 7 or higher. Preferably the amount of F-ions is less than 2000 ppm, more preferably from 100 to 1200 ppm.

The present invention provides a number of advantages, some of which are related to appearance of the toothpaste, such as the possibility to provide two or more different colours in a clear or opaque material.

In another aspect the invention provides for the possibility to physically separate various components in the toothpaste, resulting in a toothpaste containing components that would preferably be used together, but which negatively influence each other during storage. Enzyme preparations may be stored in the microspheres to avoid the degradation of thickening polymers used. The commercial carbohydrates that may be used in toothpaste's, usually contain some cellulases as contamination, which cellulases will break down cellulose gums used for thickening.

It is also possible, to include some components in the microspheres, which would otherwise require difficult or expensive production processes. An example thereof is the inclusion of abrasive agents in the microspheres, having the consequence that less, expensive, humectant is required to make a clear gel phase having a refractive index similar to the abrasive.

In a further aspect one can include components in one or more of the types of microspheres, which are only liberated after some specified time or amount of shear during brushing, thus enabling the user to determine the minimal time needed for brushing. As the strength of the microsphere wall can be regulated in the production process, microspheres containing a specific flavour may be used as a 'brushing time indicator'. Any commonly used flavouring agent will do. Those that change taste after some time, such as limoneencyclodextrine + isoamylacetate are preferred. Also colouring agents can be used, preferably those changing colour after some time. Suitable colouring agents are phenolphtalein, methylene blue, chlorophenol red and chlorophyl. It is to be noted that incorporation of those reactive flavouring and coloring agents in the microspheres will prevent them from reacting already during storage. Additionally the effect may be two-fold, first the agent being set free from the millispheres and subsequently changing character.

The amount of microspheres in the toothpaste according to the invention preferably ranges from 0.1 to 40 vol.%.

Other active ingredients that may be incorporated in the microspheres are bactericides, such as chlorhexidine and lantibiotics such as nisin, antitartar compounds such as zinc chloride, anti caries compounds such as aminfluorides, and other compounds that would otherwise be inactivated by the conventional dentifrice ingredients.

The toothpaste's according to the invention may contain the conventional components that are usually present in those products. The choice thereof depends on the actual type of product.

Toothpaste's generally contain polishing material. Examples of polishing materials are water-insoluble sodium metaphosphate, potassium metaphosphate, tricalcium phosphate, dehydrated calcium phosphate, anhydrous dicalcium phosphate, calcium pyrophosphate, magnesium orthophosphate, trimagnesium phosphate, aluminium oxide, aluminium oxide hydrate, sodium bicarbonate, calcium carbonate, aluminium silicate, zirconium silicate, silica, bentonite, and mixtures thereof. Other suitable polishing materials include the particulate thermosetting resins described in U.S. Patent No. 3 070 510, such as melamine-phenolic-, and urea-formaldehydes, and cross-linked polyepoxides and polyesters. Preferred polishing materials include silica's, such as colloidal silica, and complex amorphous alkali metal aluminosilicate.

When visually clear or opaque gels are desired, a polishing agent of colloidal silica, such as those sold under the trademark SYLOID as Syloid 72 and Syloid 74 or under the trademark SANTOCEL as Santocel 100 and alkali metal alumino-silicate complexes are particularly useful, since they have refractive indices close to the refractive indices of gelling agent-liquid (including water and/or humectant) systems commonly used in dentifrice's.

The polishing material is generally present in the solid or paste compositions in weight concentrations of about 10% to about 75%.

In a toothpaste, the liquid vehicle may comprise water and humectant typically in an amount ranging from about 10% to about 90% by weight of the preparation. Glycerine, propylene glycol, sorbitol, polypropylene glycol and/or polyethylene glycol exemplify suitable humectants/carriers. Also advantageous are liquid mixtures of water, glycerine and sorbitol. In clear gels where the refractive index is an important consideration, about 3-30 wt% of water, 0 to about 80 wt% of glycerine, and about 20-80 wt% of sorbitol is preferably employed.

Toothpaste's and gels typically contain a natural or synthetic thickener or gelling agent in proportions of about 0.1 to about 10, preferably about 0.5 to about 5 wt%. A suitable thickener is synthetic hectorite, a synthetic colloidal magnesium alkali metal silicate complex clay available for example as Laponite (e.g. CP, SP 2002, D) marketed by Laporte Industries Limited.

Other suitable thickeners include Irish moss, gum tragacanth, starch, polyvinylpyrrolidone, hydroxyethylpropyl cellulose, hydroxybutyl methyl cellulose, hydroxypropyl methyl cellulose, hydroxyethyl cellulose (e.g. available as Natrosol), sodium carboxymethyl cellulose, colloidal silica such as finely ground Syloid, and carraghene.

Any suitable flavouring or sweetening material may also be employed. Examples of suitable flavouring constituents are flavouring oils, e.g. oil of spearmint, peppermint, wintergreen, sassafras, clove, sage, eucalyptus, marjoram, cinnamon, lemon, and orange, and methyl salicylate. Suitable sweetening agents include sucrose, lactose, maltose, sorbitol, xylitol, sodium cyclamate, perillartine, APM (aspartyl phenyl alanine methyl ester), saccharine and the like. Suitably, flavour and sweetening agents may together comprise from about 0.1% to 5% or more of the preparation.

Other active components may be present, such as phosphates, such as trimetaphosphates and diammoniumphosphates, enzymes, anti-tartar agents such as pyrophosphates, zinc-compounds and phosphonates and/or other anti-plaque ingredients, such as triclosan, chlorhexidine, bromochlorophene and sanquinarine, agents for sensitive teeth, such as specific alkalimetal salts like strontium salts and/or potassium nitrate or - citrate, wound healing agents such as allantoin, chlorophyll, tocopherol and herbal extracts, activity enhancing agents, or boosters, as gantrez and some polymers, and specific ingredients as urea, xylitol, silicones, quaternary ammonium compounds and mixtures thereof. These components, as well as the amounts to be used, are known in the art. They are, where present, incorporated in the preparations in amounts which do not substantially adversely affect the properties and characteristics desired.

Examples of suitable additional anti-plaque agents are triclosan, chlorhexidine, bromochlorophene, sanquinarine, metal salts and xylitol. Anti-calculus agents can for example be chosen from pyrophosphate, zinc-compounds and diphosphonates.

Agents for sensitive dentition are usually specific alkalimetal salts, like strontium salts and potassium nitrate, or -citrate. Wound healing agents can for example be allantoin, chlorophyl, tocopherol and herbal extracts. Finally the activity enhancing agents, or boosters, can be present to enhance the delivery and retention of the active components. Examples are Gantrez® and some polymers.

The use of microspheres according to the present invention can advantageously be combined with the use of the enzyme systems that are disclosed in US patents Nos. 4 150 113 and 4 871 532. According to these patents hydrogen peroxide producing enzyme systems have advantageous effects on the reduction of oral bacterial activity. The most effective enzyme is the oxidoreductase enzyme glucose oxidase. This enzyme may be present in combination with other enzymes such as amyloglyclosidase, dextranase and/or mutanase, optionally in the presence of zinc ion providing compounds and/or 8-hydroxyquinoline derivatives. Other enzymes like those disclosed in US patent 4 152 418 may also be present.

Other enzymes like lactoperoxidase, lactoferrin and lysozyme may also be present and provide a toothpaste having advantageous properties.

A toothpaste according to the present invention may additionally contain an effective amount of a bactericide, such as lantibiotic, for example nisin or subtillin.

Preferably the microspheres in the toothpaste according to the invention contain at least one active ingredient selected from flavours, colorants, polishing agents, abrasive agents, enzymes, sweeteners, humectants, disinfecting agents, thickening agents, sweetening agents, foaming agents, bactericidal agenst and/or other active components.

The toothpaste's according to the present invention can be prepared by the conventional ways of preparing said mouth-care products. The microspheres can be added to the finished toothpaste or can be added during the production process, which is preferred. The process parameters like temperatures and pressures may have the conventional values.

The pH of the dentifrice preparation of the invention is generally in the range of from about 4.5 to about 10 and typically from about 5.5. to 9.

The pH is preferably in the range of from about 5.5 to about 8.0. It is noteworthy that the compositions of the invention may be applied orally at the said pH ranges without substantially decalcifying or otherwise damaging dental enamel. The pH can be controlled with acid (e.g. citric acid or benzoic acid) or base (e.g. sodium hydroxide) or be buffered (e.g. with sodium citrate, benzoate, carbonate, or bicarbonate, disodium hydrogen phosphate, or sodium dihydrogen phosphate).

The process for preparing the microspheres to be used in the toothpaste according to the invention comprises providing a solution of a suitable zinc salt in water and letting droplets of an alginate solution in water, optionally containing further components fall into the said zinc-ions containing solution.

Preferably the said alginate solution further contains at least one surface active compound, for example poly-ethylene glycol, or ethoxylated glycerol. The alginate solution should also comprise the active components that have to be incorporated in the microspheres, as discussed previously.

The actual process conditions for preparing the microspheres can be selected by a skilled artisan by routine experimentation, based on the required properties of the microspheres. By selecting concentration of zinc, alginate and the additives to be incorporated in the microspheres, the structure and chemical composition of the spheres is determined. The temperature, rate of stirring, but also concentration of various components determine the size of the spheres.

Temperatures can be selected within wide ranges, whereby it is preferred to use temperatures below 95°C, more in particular between room temperature (20°C) and 40°C. The concentration of zinc ions in the aqueous solution is preferably at least 0.01 M and preferably between 0.05 and 0.5 M.

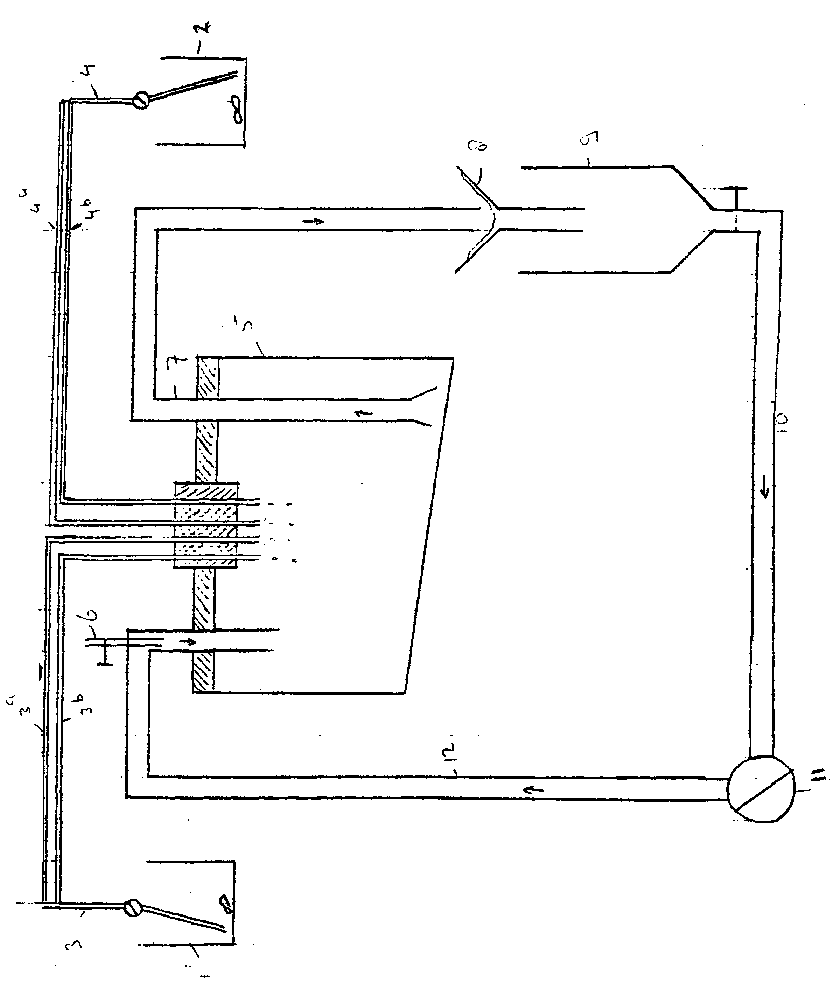

In the figure a schematic diagram of a process for preparing the microspheres is disclosed. In this diagram two containers 1,2 for the alginate solution are shown. The contents of both containers is continuously stirred. The two solutions are each fed through lines 3, 3^{a} and 3^{b}, respectively lines 4, 4^{a} and 4^{b}, to reaction vessel 5. The use of two feed lines per container increases the flexibility and control of particle size.

Reaction vessel 5 is filled with a zinc chloride solution of suitable concentration, the amount and concentration of which can be corrected, if necessary, via line 6.

When leaving the feed lines, the alginate solution falls into the reaction mixture in the reaction vessel in the form of droplets. Due to the alginate-zinc interaction the droplets are converted into microspheres, which sink to the bottom of vessel 5 and are entrained by the recirculating solution through pipe 7. The recirculating solution is fed to funnel 8 and buffer vessel 9. Via line 10 the microsphere containing solution is fed to sieve 11, where the microspheres are recovered. The residual, microsphere-free zinc chloride solution is returned to vessel 5 through line 12.

The invention will now be illustrated by means of the following examples. These examples are only used by way of illustration and are not intended to restrict the scope of the invention.

### Example 1

Using a process as described in relation to the figure microspheres were prepared.

First a sodium alginate solution was prepared by dissolving 0.1 g sodium methyl paraben in 90 g hot water (70°C), adding this solution while stirring to a mixture of 10 g PEG 600 and 1.0 g sodium alginate. After cooling to 35°C colouring agent was added to the solution.

A 0.1M zinc chloride solution having a temperature of 30°C was provided in the container, to which the solution of PEG 600, sodium alginate, sodium methyl paraben and colouring agent was added dropwise.

The microspheres are formed directly upon contact of the droplets and the water. They are removed from the container, washed and stored under water.

### Example 2

A toothpaste was prepared using the following ingredients, using known techniques for preparing toothpastes.

| Component | Parts by weight |
|---|---|
| Water | 5.61 |
| sorbitol (70% aq. solution) | 59.21 |
| sodium monofluorphosphate | 0.80 |
| Na₂HPO₄.2H₂O | 0.30 |
| Na₃PO₄ | 0.48 |
| Sodium saccharin | 0.20 |
| Na C₁₄₋₁₆ olefin sulfonate (38% aq.solution) | 3.45 |
| hydrated silica | 18.00 |
| PEG 600 | 7.00 |
| Xanthan gum | 0.70 |
| Flavour | 0.90 |

15 parts by weight of the microspheres prepared in accordance with Example 1 were added during the production of the toothpaste, after the mixing of the first seven components and prior to the addition of abrasive agent and the thickeners.

### Example 3

A toothpaste was prepared in the same manner as in Example 2, having the following composition.

| Component | Parts by weight |
|---|---|
| Water | 43.35 |
| sorbitol (70% aq. solution) | 43.00 |
| Carbopol 940 | 1.00 |
| PVP | 2.00 |
| Sodium saccharin | 0.10 |
| Na methyl paraben | 0.20 |
| NaOH (10%) | 4.00 |
| Propylene glycol | 3.00 |
| Xanthan gum | 0.70 |
| Ethoxylated stearyl alcohol | 2.00 |
| Flavour | 0.20 |
| Microspheres | 10.00 |

## Claims

1. Mouth-care product in the form of a toothpaste comprising microspheres having a particle size in the range of 0.1 to 4 mm, said microspheres being based on zinc-stabilised alginate.

2. Toothpaste according to claim 1, wherein the said microspheres contain at least one pigment.

3. Toothpaste according to claim 2, wherein at least two types of microspheres are present with different colours.

4. Toothpaste according to claims 1-3, wherein the amount of microspheres in the toothpaste ranges from 0.1 to 40 vol.%.

5. Toothpaste according to claims 1-4, which contains substantially no uncomplexed calcium.

6. Toothpaste according to claim 5, wherein calciumcitrate is present as calcium source in the toothpaste.

7. Toothpaste according to claims 1-6, wherein the amount of F- ions is less than 2000 ppm.

8. Toothpaste according to claims 1-7, wherein the F- content ranges from 100 to 1200 ppm.

9. Toothpaste according to claim 8, wherein the said F- content is provided by MFP.

10. Toothpaste according to claim 9, wherein the pH of the toothpaste is 7 or higher.

11. Toothpaste according to claims 1-10, wherein the microspheres contain at least one active ingredient.

12. Toothpaste according to claim 11, wherein the active ingredient is selected from flavours, colorants, polishing agents, abrasive agents, enzymes, sweeteners, humectants, disinfecting agents, thickening agents, sweetening agents, foaming agents, bactericidal agents and/or other active components.

13. Toothpaste comprising at least two types of microspheres having a particle size in the range of 0.1 to 4 mm and having different composition.

## Patentansprüche

1. Mundpflegeprodukt in Form einer Zahnpasta, umfassend Mikrokügelchen mit einer Partikelgröße im Bereich von 0,1 bis 4 mm, wobei die Mikrokügelchen auf Zinkstabilisiertem Alginat basieren.

2. Zahnpasta nach Anspruch 1, wobei die Mikrokügelchen mindestens ein Pigment enthalten.

3. Zahnpasta nach Anspruch 2, wobei mindestens zwei Typen Mikrokügelchen mit verschiedenen Farben vorhanden sind.

4. Zahnpasta nach den Ansprüchen 1 bis 3, wobei die Menge der Mikrokügelchen in der Zahnpaste von 0,1 bis 40 Vol.-% reicht.

5. Zahnpasta nach den Ansprüchen 1 bis 4, welche im wesentlichen kein unkomplexiertes Calcium enthält.

6. Zahnpasta nach Anspruch 5, wobei Calciumcitrat als Calciumquelle in der Zahnpasta vorhanden ist.

7. Zahnpasta nach den Ansprüchen 1 bis 6, wobei die Menge an F-Ionen geringer ist als 2000 ppm.

8. Zahnpasta nach den Ansprüchen 1 bis 7, wobei der F-Gehalt von 100 bis 1200 ppm reicht.

9. Zahnpasta nach Anspruch 8, wobei der F-Gehalt durch MFP bereitgestellt wird.

10. Zahnpasta nach Anspruch 9, wobei der pH der Zahnpasta 7 oder höher ist.

11. Zahnpasta nach den Ansprüchen 1 bis 10, wobei die Mikrokügelchen mindestens einen aktiven Bestandteil enthalten.

12. Zahnpasta nach Anspruch 11, wobei der aktive Bestandteil ausgewählt wird aus Geschmacksstoffen, Farbstoffen, Poliermitteln, Abrasivstoffen, Enzymen, Süßstoffen, Feuchthaltemitteln, Desinfektionsmitteln, Verdickungsmitteln, Süßungsmitteln, Schäumungsmitteln, bakteriziden Mitteln und/oder weiteren aktiven Komponenten.

13. Zahnpasta, umfassend mindestens zwei Typen Mikrokügelchen mit einer Partikelgröße im Bereich von 0,1 bis 4 mm, welche unterschiedliche Zusammensetzungen aufweisen.

## Revendications

1. Produit pour les soins buccaux sous la forme d'un dentifrice comprenant des microsphères présentant une taille de particule dans la plage de 0,1 à 4 mm, lesdites microsphères étant à base d'alginate stabilisé par du zinc.

2. Dentifrice selon la revendication 1, dans lequel lesdites microsphères contiennent au moins un pigment.

3. Dentifrice selon la revendication 2, dans lequel au moins deux types de microsphères de couleurs différentes sont présents.

4. Dentifrice selon les revendications 1 à 3, dans lequel la proportion de microsphères dans le dentifrice s'étend de 0,1 à 40 % en volume.

5. Dentifrice selon les revendications 1 à 4, lequel ne contient pratiquement pas de calcium non complexé.

6. Dentifrice selon la revendication 5, dans lequel du citrate de calcium est présent en tant que source de calcium dans le dentifrice.

7. Dentifrice selon les revendications 1 à 6, dans lequel la quantité d'ions F⁻ est inférieure à 2000 ppm.

8. Dentifrice selon les revendications 1 à 7, dans lequel la teneur en F⁻ s'étend de 100 à 1200 ppm.

9. Dentifrice selon la revendication 8, dans lequel ladite teneur en F⁻ est apportée par du MFP.

10. Dentifrice selon la revendication 9, dans lequel le pH du dentifrice est de 7 ou plus.

11. Dentifrice selon la revendication 1 à 10, dans lequel les microsphères contiennent au moins un principe actif.

12. Dentifrice selon la revendication 11, dans lequel le principe actif est choisi parmi des arômes, des colorants, des agents de polissage, des agents abrasifs, des enzymes, des adoucissants, des humectants, des agents désinfectants, des agents épaississants, des agents adoucissants, des agents de moussage, des agents bactéricides et/ou d'autres composants actifs.

13. Dentifrice comprenant au moins deux types de microsphères présentant une taille de particule dans la plage de 0,1 à 4 mm et présentant des compositions différentes.
